Europäisches Patentamt

European Patent Office

Office européen des brevets

(11) Numéro de publication: **0 242 259 B1**

## (12) FASCICULE DE BREVET EUROPEEN

(45) Date de publication de fascicule du brevet:
21.11.91

(51) Int. Cl.5: **A61K 35/50**, A61K 37/22

(21) Numéro de dépôt: 87400715.6

(22) Date de dépôt: 01.04.87

(54) Procédé de préparation de lipides riches en acides gras polyinsaturés à longue chaîne.

(30) Priorité: 14.04.86 FR 8605269

(43) Date de publication de la demande:
21.10.87 Bulletin 87/43

(45) Mention de la délivrance du brevet:
21.11.91 Bulletin 91/47

(84) Etats contractants désignés:
AT BE CH DE ES GB GR IT LI NL SE

(56) Documents cités:
EP-A- 0 082 818
EP-A- 0 132 178

NATURE, vol. 217, 27 janvier 1968, pages 378-379, McMillan Journals, Basingstoke, GB; A. ROBERTSON et al.: "Free fatty acid patterns of human maternal plasma, perfused placenta and umbilical cord plasma"

(73) Titulaire: **PASTEUR MERIEUX SERUMS ET VACCINS**
58, avenue Leclerc
F-69007 Lyon(FR)

(72) Inventeur: **Chirouze, Véronique**
40 Chemin des Fonds
F-69 110 Ste-Foy-Les Lyon(FR)
Inventeur: **Tayot, Jean-Louis**
1 rue des Greffières
F-69 890 la Tour de Salvagny(FR)

(74) Mandataire: **Lemoine, Michel et al**
Cabinet Michel Lemoine et Bernasconi 13
Boulevard des Batignolles
F-75008 Paris(FR)

Rank Xerox (UK) Business Services

## Description

La présente invention a trait à un procédé de préparation de lipides riches en acides gras polyinsaturés à chaîne longue et particulièrement en acides arachidonique C 20:4 n-6 (ARA), di-homo-gamma-linolénique C 20:3 n-6 (DHL) et docosahexaénoïque C 22:6 n-3 (DHA).

Les acides gras polyinsaturés à chaîne longue et, notamment, les acides ARA, DHL ET DHA sous forme libre ou conjuguée, constituent des compléments nutritifs particulièrement intéressants pour des préparations diététiques administrées par voie orale ou par sonde. En effet, ces nutriments trouvent des applications dans les traitements de carences en acides gras essentiels liés à certains phénomènes pathologiques tels que notamment des troubles métaboliques situés principalement au niveau des désaturases (enzymes limitantes de la biosynthèse des acides gras essentiels à partir de l'acide linoléique), de tels troubles étant observés, notamment, chez des enfants prématurés, chez des personnes âgées et chez les sujets soumis de façon prolongée à une alimentation artificielle ou encore chez les enfants atteints de mucoviscidose. Les sources généralement utilisées pour obtenir des préparations riches en de tels lipides, par exemple le foie de porc, sont relativement onéreuses.

C'est pourquoi on a déjà proposé de préparer des extraits riches en acide arachidonique à partir du placenta. Ainsi la demande de brevet français 2 553 261 déposée le 14.10.1983 décrit un lait artificiel pour l'alimentation de nouveaux-nés, caractérisé en ce qu'il contient au moins les phospholipides d'un extrait lipidique de placenta, de telle sorte que la teneur en acide arachidonique soit de l'ordre de 1 %. Ce document ne précise cependant pas de quelle partie placentaire les lipides se trouvent extraits.

Le demande de brevet européen 0 082 818, déposée le 15 décembre 1982, décrit un procédé pour l'obtention de lipides d'origine humaine et du lacto-N-norhexaosyl céramide obtenus à partir du placenta, convenablement broyé. L'extraction est effectuée au chloroforme-méthanol.

Un tel procédé présente cependant l'inconvénient de ne pas permettre l'extraction simultanée des protéines du sang placentaire telles que les immunoglobulines, l'albumine, l'hémoglobine ou la globine.

La présente invention se propose de remédier à ces inconvénients et de fournir un procédé de préparation d'acides polyinsaturés à chaîne longue et notamment d'acides ARA, DHL et DHA, en particulier d'origine humaine, à concentration élevée et à un prix de revient abaissé.

Un autre objectif de l'invention est de fournir un tel procédé permettant la préparation simultanée des autres produits de haute valeur ajoutée présents dans le placenta et notamment les IgG, l'albumine, l'hémoglobine, la globine et les protéines du sang placentaire en général.

Un autre objectif encore de l'invention est de fournir un procédé de préparation permettant de réaliser des fractions ayant une teneur particulièrement élevée en acide arachidonique, y compris sous forme libre ou ester éthylique.

La présente invention a pour objet un procédé de préparation d'extraits placentaires riches en acides gras polyinsaturés à chaîne longue, notamment en acides ARA, DHL et DHA, caractérisé en ce que l'on sépare le sang d'origine placentaire d'avec le tissu placentaire et que l'on extrait au moins une fraction lipidique de ce sang.

De façon particulièrement avantageuse, on peut procéder à l'extraction, non pas du liquide sanguin obtenu directement après séparation d'avec le tissu placentaire, mais de fractions résultant d'opérations de séparation de constituants, notamment protéiques, du sang placentaire. On peut ainsi, grâce à l'invention, obtenir des quantités notables en acides gras désirés et, notamment, en acides ARA, DHA et DHL, tout en séparant et purifiant les constituants intéressants du sang et, notamment, l'albumine, les gammaglobulines, l'hémoglobine et la globine.

Dans un premier mode de mise en oeuvre de l'invention, on prépare une fraction riche en globulines par un procédé de fractionnement alcoolique, du type méthode de Cohn ou de Taylor, de façon à former un précipité contenant les globulines. Le précipité est remis en suspension et la fraction restant insoluble est recueillie, par exemple par centrifugation, le surnageant étant traité de façon usuelle pour la préparation des globulines.

On procède ensuite à l'extraction de la fraction lipidique du culot de centrifugation. Il est ainsi possible d'obtenir une quantité notable d'acides ARA, DHL et DHA.

Dans un second mode de mise en oeuvre de l'invention, qui peut d'ailleurs être cumulé avec le premier, le surnageant de l'étape initiale d'un procédé de précipitation alcoolique, qui contient l'albumine ainsi que l'hémoglobine, est soumis à une précipitation, par exemple au chloroforme, permettant de précipiter l'hémoglobine placentaire.

On procède ensuite à l'extraction de la fraction lipidique à partir de ce précipité contenant l'hémoglobine placentaire et l'on obtient également une quantité notable d'acides ARA, DHL et DHA.

Dans un troisième mode de mise en oeuvre, qui permet de récupérer sous forme non dénaturée l'albumine, l'hémoglobine et, si on le désire, les gamma-globulines , on soumet le liquide sanguin à une étape de clarification préalable, de préférence à pH acide, permettant d'obtenir un précipité, le surnageant pouvant ensuite être soumis à une chromatographie sur support chromatographique échangeur d'anions permettant de séparer l'albumine et le mélange immunoglobulines-hémoglobine sous forme non dénaturée.

Le précipité provenant de l'étape de clarification est alors soumis à une étape d'extraction lipidique permettant d'obtenir un extrait riche en acides ARA, DHL et DHA.

L'extraction de la fraction lipidique riche en acides gras polyinsaturés à longue chaîne peut avantageusement être effectuée par les moyens classiques déjà décrits : extraction par solvants à l'état liquide (mélanges hexane-isopropanol ou chloroforme-méthanol) ou à l'état supercritique (dioxyde de carbone).

Les fractions riches en acides gras polyinsaturés à longue chaîne sont obtenues par le procédé selon l'invention.

Ces extraits se caractérisent notamment par une teneur très élevée en acide arachidonique pouvant facilement être supérieure à 25 % de la masse des acides gras présents ; par une teneur en acide DHL pouvant être comprise entre 2 et 12 % de la masse des acides gras présents ; par une teneur en acide DHA pouvant être comprise entre 1 et 6 % de la masse des acides gras présents.

D'autres avantages et caractéristiques de l'invention apparaîtront à la lecture de la description suivante.

Exemple 1.

Quinze tonnes de placenta congelé sont broyées et décongelées à 10°C dans un volume de 10 m³ de solution contenant 6 g/l de chlorure de sodium à 8 % d'alcool éthylique. Après élimination des tissus par pressage, le bloc de globulines est précipité en 24 heures d'après la méthode de Taylor à pH neutre et à -5°C par addition d'alcool jusqu'à un taux de 25 % environ. L'albumine reste dans le surnageant.

Le précipité de globulines est recueilli par centrifugation et il est ensuite dispersé à pH 4,8 dans environ 10 000 litres d'une solution contenant 4 g/l de chlorure de sodium, à une température de 2°C.

La fraction insoluble forme une suspension d'environ 1.200 litres, appelée ci-après précipité B1, et recueillie par centrifugation et le surnageant qui contient les gamma-globulines est soumis à un fractionnement ultérieur permettant la préparation des gamma-globulines.

Le précipité B1 est soumis à une extraction par 18 volumes du mélange hexane-isopropanol (3:2). La phase supérieure est lavée par une solution aqueuse de sulfate de sodium.

On obtient ainsi une fraction lipidique particulièrement riche en acides gras polyinsaturés à chaîne longue et notamment en acides ARA, DHL et DHA.

La composition de cette fraction lipidique est précisée dans le tableau 1 ci-dessous.

Tableau 1 : Composition en principaux acides gras des fractions lipidiques du précipité B1.

| Nature de l'acide gras | Phospholipides | Acides gras libres | Esters éthyliques |
|---|---|---|---|
| 15:0 | 0,2 | 0,3 | 0,5 |
| 16:0 | } 34,0 | 32,3 | 8,7 |
| 16:1 | | 3,0 | 3,7 |
| 18:0 | 15,6 | 13,6 | 4,0 |
| 18:1 n-9 | 12,0 | 20,0 | 15,8 |
| 18:2 n-6 | 8,5 | 10,3 | 15,2 |
| 20:2 n-6 | 0,5 | 0,6 | 1,0 |
| 20:3 n-6 | 3,8 | 2,7 | 8,0 |
| 20:4 n-6 | 13,6 | 10,9 | 33,6 |
| 22:4 n-6 | 1,3 | 0,9 | 1,5 |
| 22:5 n-6 | 0,7 | 0,9 | 0,7 |
| 22:5 n-3 | 1,5 | 0,4 | 0,9 |
| 22:6 n-3 | 3,8 | 3,5 | 2,0 |

Légende des produits soulignés
20:3 n-6      Acide di homo gamma linoléique
20:4 n-6      Acide arachidonique
22:6 n-3      Acide docosa hexaénoïque

On peut ainsi obtenir environ 130 grammes d'acide arachidonique par tonne de placenta, cet acide arachidonique (20:4 n-6) se trouvant disponible dans les fractions lipidiques majeures, à savoir les phospholipides, les acides gras libres et, en quantité particulièrement importante, sous forme d'ester éthylique, ce qui est particulièrement surprenant puisque l'acide arachidonique, qui se trouve en position 2 dans les phospholipides, ne paraît pas pouvoir être hydrolysé en l'absence d'un traitement enzymatique approprié.

Exemple 2.

Le surnageant de la précipitation de Taylor de l'exemple 1, qui contient notamment l'albumine et l'hémoglobine du liquide sanguin placentaire, est soumis à une précipitation au chloroforme à 5,4 g/1 à une température d'environ 20°. On effectue ensuite une filtration sous vide sur filtre rotatif permettant de récupérer 4 tonnes par jour de précipité contenant l'hémoglobine placentaire.

Ce précipité est soumis à une extraction par 18 volumes du mélange hexane-isopropanol (3:2). La phase supérieure est lavée par une solution aqueuse de sulfate de sodium.

La composition en acides gras de l'extrait obtenu apparaît sur le tableau 2 ci-dessous.

Tableau 2

| Composition en principaux acides gras des fractions lipidiques du précipité hémoglobine placentaire. | | |
|---|---|---|
| Nature de l'acide gras | Phospholipides | Acides gras libres |
| 15:0 | - | - |
| 16:0 | 26,4 | 12,6 |
| 16,1 | 1,8 | 3,2 |
| 18,0 | 13,2 | 5,1 |
| 18:1 n-9 | 11,2 | 17,6 |
| 18:2 n-6 | 10,5 | 18,1 |
| 20:1 | - | - |
| 20:2 n-6 | 0,5 | 0,5 |
| 20:3 n-6 | 4,6 | 5,6 |
| 20:4 n-6 | 18,2 | 28,7 |
| 22:4 n-6 | 1,1 | 1,3 |
| 22:5 n-6 | 0,8 | 1,1 |
| 22:5 n-3 | 0,8 | 0,8 |
| 22:6 n-3 | 4,0 | 5,5 |

On voit là également que l'on obtient une quantité particulièrement importante d'acide arachidonique, notamment dans la fraction des acides gras libres.

On peut ainsi extraire de 90 à 98 grammes d'acide arachidonique par tonne de placenta.

Exemple 3.

Le liquide sanguin séparé du tissu placentaire par presssage comme dans l'exemple 1 est d'abord soumis à une étape de clarification dans laquelle le liquide sanguin est additionné d'acide acétique ou chlorhydrique N, indifféremment, pour ajuster le pH à 5,10 sous agitation à 0°C. Après deux heures d'attente, la suspension est injectée à 300 l/heure sur centrifugeuse. On obtient ainsi un précipité de l'ordre de 7 à 10 kg pour 150 kg de placenta humain. Le surnageant est ensuite traité pour la séparation de l'albumine ou de l'hémoglobine native comme décrit dans le brevet français n° 83-11323 du 7 juillet 1983.

Le précipité provenant de l'étape de clarification, intitulé "nouveau précipité B1", est soumis à une extraction par 18 volumes du mélange hexane-isopropanol 3:2). La phase supérieure est lavée par une solution aqueuse de sulfate de sodium.

La composition en acide gras des principales fractions lipidiques de l'extrait est représentée dans le tableau 3 ci-dessous.

Tableau 3 : Composition en principaux acides gras des fractions lipidiques du nouveau précipité B1.

| Nature de l'acide gras | Phospholipides | Acides gras libres | Esters éthyliques |
|---|---|---|---|
| 15:0 | 0,2 | 0,7 | 0,2 |
| 16:0 | } 31,8 | 20,4 | 5,1 |
| 16:1 | | 4,0 | 1,5 |
| 18:0 | 15,0 | 11,8 | 2,0 |
| 18:1 n-9 | 12,4 | 17,8 | 8,4 |
| 18:2 n-6 | 10,2 | 17,7 | 12,5 |
| 20:2 n-6 | 0,3 | 0,2 | 0,3 |
| 20:3 n-6 | 3,6 | 3,4 | 11,4 |
| 20:4 n-6 | 15,7 | 11,7 | 52,5 |
| 22:4 n-6 | 1,6 | 1,4 | 1,5 |
| 22:5 n-6 | 1,0 | 1,5 | 0,5 |
| 22:5 n-3 | 0,7 | 0,4 | 0,8 |
| 22:6 n-3 | 2,8 | 1,3 | 1,4 |

On remarque que la fraction ester éthylique est plus riche en acide 20:3 n-6 et 20:4 n-6 que la fraction décrite dans l'exemple 1 et correspondant au précipité B1.

On peut ainsi, à partir du "nouveau précipité B1", obtenir 160 grammes environ d'acide arachidonique par tonne de placenta mise en oeuvre.

En résumé, l'invention montre qu'il est possible de produire des quantités importantes de lipides placentaires et notamment de lipides polyinsaturés à chaîne longue (ARA, DHL et DHA) en particulier de 160 à 220 grammes d'acide arachidonique par tonne de placenta, et ceci en permettant simultanément d'obtenir les fractions protéiques, notamment les globulines, l'albumine, l'hémoglobine et la globine, sans perte quantitative ni qualitative au niveau de ces fractions.

**Revendications**

1. Procédé de préparation d'extraits placentaires riches en acides gras polyinsaturés à chaîne longue, notamment en acides ARA, DHL et DHA, caractérisé en ce que l'on sépare le sang d'origine placentaire d'avec le tissu placentaire, que l'on soumet le sang placentaire à un fractionnement pour la séparation de constituants, notamment protéiques, du sang placentaire, et en ce que l'on extrait au moins une fraction lipidique de ce sang placentaire.

2. Procédé selon la revendication 1, caractérisé en ce que l'on prépare une fraction riche en globuline par un procédé de fractionnement alcoolique formant un précipité en suspension et que l'on recueille la fraction restant insoluble, après quoi on procède à l'extraction des lipides de ladite fraction.

3. Procédé selon l'une quelconque des revendications 1 et 2, caractérisé en ce que l'on prépare une fraction riche en albumine et hémoglobine par un procédé de fractionnement alcoolique et que l'on procède à une précipitation de l'hémoglobine, après quoi on procède à l'extraction de la fraction lipidique du précipité contenant l'hémoglobine placentaire.

4. Procédé selon la revendication 3, caractérisé en ce que le surnageant contenant l'albumine et l'hémoglobine est soumis à une précipitation au chloroforme.

5. Procédé selon la revendication 1, caractérisé en ce que l'on soumet le liquide sanguin à une étape de clarification permettant d'obtenir un précipité et que l'on soumet ledit précipité à une étape d'extraction lipidique.

6. Procédé selon la revendication 5, caractérisé en ce que l'étape de clarification est effectuée à pH acide.

7. Procédé selon l'une quelconque des revendications 1 à 6, caractérisé en ce que l'on effectue l'extraction de la fraction lipidique par un mélange de solvants dans l'état liquide et/ou supercritique.

**Claims**

1. A process for the preparation of placental extracts rich in long-chain poly-unsaturated fatty acids, notably in the acids ARA, DHL, and DHA, characterized in that blood of placental origin is separated from placental tissue, in that placental blood is subjected to a fractionation for the separation of constituents, notably proteinic, of the placental blood and in that at least one lipid fraction is extracted from this placental blood.

2. The process according to claim 1, characterized in that a fraction rich in globulin is prepared by a process of alcohol fractionation forming a precipitate in suspension and the fraction remaining insoluble is collected, after which the extraction of the lipids from the said fraction is undertaken.

3. The process according to any one of the claims 1 or 2, characterized in that a fraction rich in albumin and hemoglobin is prepared by a process of alcohol fractionation and a precipitation of the hemoglobin is carried out, after which the extraction of the lipid fraction from the precipitate containing the placental hemoglobin is undertaken.

4. The process according to claim 3, characterized in that the supernatant containing the albumin and hemoglobin is subjected to a precipitation with chloroform.

5. The process according to claim 1, characterized in that the bloody liquid is subjected to a clarification step permitting the obtaining of a precipitate and in that the said precipitate is submitted to a lipid extraction step.

6. The process according to claim 5, characterized in that the clarification step is carried out at an acid pH.

7. The process according to any one of the claims 1 to 6, characterized in that an extraction of the lipid fraction is carried out using a mixture of solvents in the liquid and/or supercritical state.

**Patentansprüche**

1. Verfahren zur Herstellung von Planzentaextrakten, die reich an mehrfachungesättigten Fettsäuren mit langer Kette sind, insbesondere an ARA-, DHL- und DHA-Säuren, **dadurch gekennzeichnet,** daß man das Blut der Plazenta vom Plazentagewebe trennt, daß man das Plazentrablut einer Fraktionierung für die Abtrennung von Bestandteilen, insbesondere von Proteinbestandteilen aus dem Plazentablut, unterwirft, und daß man wenigstens eine Lipidfraktion aus diesem Plazentablut extrahiert.

2. Verfahren nach Anspruch 1, dadurch gekennzeichnet, daß man eine an Globulin reiche Fraktion durch ein alkoholisches Fraktionierverfahren erstellt, das einen Niederschlag in Suspension bildet, und daß man die unlösliche Fraktion auffängt, worauf man mit der Extraktion der Lipide aus der genannten Fraktion fortfährt.

3. Verfahren nach einem der Ansprüche 1 und 2, dadurch gekennzeichnet, daß man eine an Albumin und Hämoglobulin reiche Fraktion durch ein alkoholisches Fraktionierverfahren herstellt und man mit einer Abscheidung des Hämoglobulins fortfährt, waraufhin man mit der Extraktion der lipidischen Fraktion des Niederschlags, der das Plazenta-Hämoglobulin enthält, fortfährt.

4. Verfahren nach Anspruch 3, dadurch gekennzeichnet, daß der oben schwimmende Bestandteil, der das Albumin und das Hämoglobulin enthält, einem Niederschlag mit Chloroform unterworfen wird.

5. Verfahren nach Anspruch 1, dadurch gekennzeichnet, daß man die Blutflüssigkeit einem Klärungsschritt unterwirft, der es erlaubt, einen Niederschlag zu erzielen, und daß man den genannten Niederschlag einer lipidischen Extraktion unterwirft.

6. Verfahren nach Anspruch 5, dadurch gekennzeichnet, daß die Klärung bei einem sauren pH-Wert durchgeführt wird.

7. Verfahren nach einem der Ansprüche 1 bis 6, dadurch gekennzeichnet, daß man die Extraktion der lipidischen Fraktion durch ein Lösungsmittelgemisch im flüssigen und/oder überkritischen Zustand ausführt.